(19) Europäisches Patentamt
European Patent Office
Office européen des brevets

(11) **EP 2 312 310 B1**

(12) **FASCICULE DE BREVET EUROPEEN**

(45) Date de publication et mention
de la délivrance du brevet:
**21.01.2015 Bulletin 2015/04**

(51) Int Cl.:
**G01N 33/24** *(2006.01)*      **G01N 30/00** *(2006.01)*
**G01V 99/00** *(2009.01)*      **G01N 25/14** *(2006.01)*

(21) Numéro de dépôt: **10306134.7**

(22) Date de dépôt: **18.10.2010**

(54) **Dispositif et procédé pour analyser un échantillon d'un minéral d'une roche**

Gerät und Verfahren zur Analyse einer Mineralprobe von einem Gestein

Device and method for analyzing a rock mineral sample

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **16.10.2009 FR 0957296**

(43) Date de publication de la demande:
**20.04.2011 Bulletin 2011/16**

(73) Titulaire: **CENTRE NATIONAL D'ETUDES
SPATIALES
75001 Paris (FR)**

(72) Inventeurs:
• **Gillot, Pierre-Yves**
**91400, ORSAY (FR)**
• **Lefevre, Jean-Claude**
**75014, PARIS (FR)**
• **Chiavassa, Florence**
**31400, TOULOUSE (FR)**

(74) Mandataire: **Blot, Philippe Robert Emile
Cabinet Lavoix
2, place d'Estienne d'Orves
75441 Paris Cedex 09 (FR)**

(56) Documents cités:
**US-A- 5 288 695**

• **SWINDLE, BODE, BOYNTON, KRING, WILLIAMS, CHUTJIAN, DARRACH, CREMERS, WIENS, BALDWIN: "AGE (Argon geochronology experiment): an instrument for in situ geochronology on the surface of Mars", 34TH LUNAR AND PLANETARY SCIENCE CONFERENCE, 2003, XP002576520,**
• **STELIOS TZORTZAKIS: "Ultraviolet laser filaments for remote laser-induced breakdown spectroscopy (LIBS) analysis: applications in cultural heritage monitoring", OPTICS LETTERS, vol. 31, no. 8, 2006, pages 1139-1141, XP002611191,**
• **CHRISTOPHER LATKOCZY, THIERRY GHISLAIN: "Simultaneous LIBS and LA-ICP-MS analysis of industrial samples", JOURNAL OF ANALYTICAL ATOMIC SPECTROSCOPY, vol. 21, 3 octobre 2006 (2006-10-03), pages 1152-1160, XP002576519,**
• **APPLICATIONS A M FENNEMA ET AL: 'A METHOD FOR FINDING THE MASS OF A MILLIGRAM-SIZED ROCK SAMPLE WITHOUT USING A SCALE, WITH POSSIBLE SPACECRAFT', [en ligne] 01 Janvier 2007, XP055126880 Extrait de l'Internet: <URL:http://www.lpi.usra.edu/meetings/lpsc2 007/pdf/1772.pdf> [extrait le 2014-07-04]**

**EP 2 312 310 B1**

**Description**

La présente invention concerne un dispositif pour analyser un échantillon d'un minéral d'une roche comprenant un nombre d'atomes d'argon 40 et une proportion en masse de potassium 40 du type comportant

- des moyens de traitement de la roche destinés à extraire un échantillon d'un minéral de la roche et à libérer l'argon 40 et le potassium naturel, qui comprend le potassium 40, de l'échantillon ;
- des premiers moyens d'analyse destinés à déterminer le nombre d'atomes d'argon 40 dans l'échantillon ; et
- des seconds moyens d'analyse destinés à déterminer la proportion en masse de potassium 40 dans l'échantillon.

Au cours de la vie d'une roche, en particulier d'une roche magmatique, le potassium 40 contenu dans la roche se désintègre pour former de l'argon 40. Les constantes de désintégration étant connues, l'âge d'une roche est déterminé en mesurant le rapport de la quantité d'argon 40 à la quantité de potassium 40 de cette roche.

Il est connu d'utiliser ce principe de la méthode potassium-argon pour dater une roche dans un laboratoire.

Ainsi, la technique la plus couramment utilisée aujourd'hui en laboratoire pour dater des roches selon cette méthode est la technique dite argon 40/ argon 39. Cette technique de mesure consiste à transformer partiellement le potassium 39 contenu dans l'échantillon en argon 39 par activation neutronique, en bombardant l'échantillon avec un flux de neutrons rapides. Le rapport de la quantité d'argon 39 à la quantité d'argon 40 radiogénique, produit dans le temps par la désintégration du potassium 40 est alors mesuré par spectrométrie de masse et l'âge de l'échantillon déduit par comparaison avec un échantillon standard d'âge connu pour lequel le même rapport est calculé alors que cet échantillon standard est soumis à un bombardement neutronique dans les mêmes conditions. Cette première technique, du fait qu'elle exige l'irradiation neutronique de l'échantillon à dater et d'un standard rocheux dans des conditions rigoureusement identiques, ne peut être utilisée dans une situation dans laquelle l'opérateur n'a pas accès directement à l'échantillon, ni la possibilité de le soumettre à un flux de neutrons.

Une deuxième méthode, plus ancienne, dite potassium 40/ argon 40 fait également appel à ce principe de datation potassium-argon. Elle consiste à mesurer les quantités respectives de potassium 40 et d'argon 40 dans deux fractions aliquotes d'un minéral de la même roche. Le potassium 40 et l'argon 40 se présentent dans des états chimiques différents, puisque le potassium 40 est à l'état solide, alors que l'argon 40 est à l'état gazeux. Des techniques d'analyse différentes sont donc utilisées pour chacun de ces éléments. Ainsi, la quantité de potassium 40 est mesurée par spectrométrie de flamme après dissolution au moyen d'une attaque acide d'une première fraction aliquote alors que la quantité d'argon 40 est déterminée en faisant fondre la deuxième fraction aliquote dans une enceinte sous vide, puis en filtrant les gaz ainsi obtenus pour isoler les gaz rares, que l'on introduit dans un spectromètre de masse après extraction de l'hélium.

Du fait de la nécessité d'extraire deux fractions aliquotes différentes d'un même minéral d'une roche et de soumettre celles-ci à des processus d'analyse différents pour obtenir respectivement la quantité de potassium 40 et la quantité d'argon 40, le dispositif expérimental permettant la mise en oeuvre de cette technique présente l'inconvénient de ne pas être facilement mécanisable et rendu pilotable à distance. Cette technique ne peut donc pas être utilisée dans une situation dans laquelle l'opérateur n'a pas accès directement à l'échantillon, celui-ci devant être analysé sur son lieu de prélèvement par un système d'analyse embarqué.

Par ailleurs, dans le cas de systèmes d'analyse embarqués dans des engins motorisés, il est très important que ces systèmes aient un poids et un encombrement aussi faibles que possible. De ce point de vue, le système d'analyse correspondant à la méthode potassium 40/ argon 40 actuellement utilisée est, du fait des nombreuses étapes de traitement requises, trop encombrant pour pouvoir être embarqué.

Les documents Swindle et. al., "AGE (Argon Geochronology expriment) : An Instrument for in situ Geochronology on the Surface of Mars", Lunar and Planetery Science XXXIV (2003), and Fennema et al., "A Method for Finding the Mass of a Milligram-Sized Rock Sample Without Using a Scale; with Possible Spacecraft applications", Lunar and Planetery Science XXXVIII (2007) décrivent un dispositif et un procédé d'analyser des échantillons de roche par la méthode K-Ar, où la masse d'un échantillon est déterminée par la mesure de la densité et du volume de l'échantillon.

La présente invention a pour but de fournir un procédé pour analyser un échantillon d'un minéral d'une roche, ainsi qu'un dispositif mettant en oeuvre ce procédé, qui peuvent être pilotés à distance, ne nécessitent pas de traitement préalable de la roche en laboratoire et ne nécessitent donc pas la présence d'un opérateur sur le lieu de prélèvement de la roche et qui présentent un encombrement réduit.

A cet effet, l'invention a pour objet un dispositif du type précité, caractérisé en ce que :

- les moyens de traitement de la roche comportent un laser destiné à tirer sur la roche pour ablater l'échantillon de la roche et former un plasma à partir de l'échantillon ;
- les premiers moyens d'analyse sont propres à analyser des gaz issus du plasma pour déterminer le nombre d'atomes d'argon 40 dans l'échantillon ;

- les seconds moyens d'analyse sont propres à analyser le plasma formé par le laser pour déterminer la proportion en masse de potassium 40 dans l'échantillon. Selon d'autres caractéristiques de l'invention :
- les premiers moyens d'analyse comprennent un spectromètre de masse ;
- les seconds moyens d'analyse comprennent un spectromètre ultraviolet ;
- le laser est destiné à émettre dans l'ultraviolet lointain ;
- le dispositif comporte en outre des moyens de visualisation de la roche, ainsi que des moyens de guidage du laser pour un tir sur une zone choisie de la roche ;
- le dispositif comporte en outre une enceinte destinée à accueillir la roche et à être mise sous vide, ainsi qu'un moyen de mise sous vide de l'enceinte ;
- le dispositif comporte en outre des moyens de séparation des gaz issus du plasma ; et
- les moyens de séparation des gaz issus du plasma comportent une pompe à sorption, ainsi qu'un dispositif de condensation des gaz.

L'invention a également pour objet un procédé d'analyse d'un échantillon d'un minéral d'une roche comprenant un nombre d'atomes d'argon 40 et une proportion en masse de potassium 40 dans l'échantillon, comportant les étapes de :

- traiter la roche à l'aide de moyens de traitement de manière à extraire un échantillon d'un minéral de la roche et à libérer l'argon 40 et le potassium naturel, qui comprend le potassium 40 de l'échantillon ;
- déterminer le nombre d'atomes d'argon 40 dans l'échantillon à l'aide de premiers moyens d'analyse ;
- déterminer la proportion en masse de potassium 40 dans l'échantillon à l'aide de seconds moyens d'analyse ;

caractérisé en ce que

- l'étape de traitement de la roche consiste à effectuer un tir sur la roche avec un laser pour ablater un échantillon d'un minéral de la roche et pour former un plasma à partir de l'échantillon ;
- on détermine le nombre d'atomes d'argon 40 dans les gaz issus du plasma formé à partir de l'échantillon à l'aide des premiers moyens d'analyse ;
- on détermine la proportion en masse de potassium 40 dans le plasma formé à partir de l'échantillon à l'aide des seconds moyens d'analyse.

L'invention a également pour objet un procédé de réalisation d'une base de données à partir d'au moins une roche de référence d'âge connu, comportant un nombre d'atomes d'argon 40, comprenant les étapes de :

- traiter la au moins une roche de référence à l'aide de moyens de traitement de manière à extraire un échantillon de référence d'un minéral de la au moins une roche de référence et à libérer l'argon 40 contenu dans l'échantillon de référence ;
- déterminer le nombre d'atomes d'argon 40 dans l'échantillon à l'aide de moyens d'analyse ;

caractérisé en ce que

- l'étape de traitement de la roche de référence consiste à effectuer un tir sur la au moins une roche de référence avec un laser pour ablater un échantillon de référence d'un minéral de la au moins une roche de référence et pour former un plasma à partir de l'échantillon ;
- on détermine le nombre d'atomes d'argon 40 dans les gaz issus du plasma formé à partir de l'échantillon de référence à l'aide des moyens d'analyse ;
- on calcule la masse de l'échantillon de référence à partir de l'âge de la au moins une roche de référence dont il est issu, de la nature du minéral qui constitue l'échantillon de référence et du nombre d'atomes d'argon 40 dans l'échantillon de référence ;
- on associe la masse de l'échantillon de référence aux caractéristiques du tir effectué par le laser et à la nature du minéral qui constitue l'échantillon de référence.

L'invention a également pour objet un procédé de détermination de l'âge d'un échantillon de roche, comportant les étapes de :

- déterminer le nombre d'atomes d'argon 40 et la proportion en masse de potassium 40 dans l'échantillon en utilisant le procédé d'analyse d'un échantillon d'un minéral d'une roche tel que défini ci-dessus ;
- réaliser une base de données associant la masse d'un échantillon de référence d'un minéral d'une roche de référence aux caractéristiques du tir du laser et à la nature du minéral constituant l'échantillon de référence en utilisant le procédé de réalisation d'une base de données tel que défini ci-dessus ;
- déterminer à l'aide de la base de données ainsi constituée la masse de l'échantillon à partir des caractéristiques du tir laser effectué pour ablater l'échantillon et de la nature du minéral constituant l'échantillon ;
- calculer l'âge de la roche à l'aide de la masse de l'échantillon, du nombre d'atomes d'argon 40 et de la proportion en masse de potassium 40 dans l'échantillon.

L'invention a également pour objet une installation pour déterminer l'âge d'une roche comportant

- un dispositif pour analyser la composition d'un échantillon d'un minéral d'une roche tel que défini ci-dessus, destiné à déterminer le nombre d'atomes d'argon 40 et la proportion en masse de potassium 40 dans l'échantillon ;
- des moyens destinés à constituer une base de données associant la masse d'un échantillon de référence d'un minéral d'une roche de référence aux caractéristiques du tir du laser et à la nature du minéral constituant l'échantillon de référence selon le procédé de réalisation d'une base de données tel que défini ci-dessus ;
- des moyens destinés à déterminer, à l'aide de la base de données ainsi constituée, la masse de l'échantillon à partir des caractéristiques du tir laser effectué pour ablater l'échantillon et de la nature du minéral constituant l'échantillon ;
- des moyens destinés à calculer l'âge de la roche à partir de la masse de l'échantillon, du nombre d'atomes d'argon 40 et de la proportion en masse de potassium 40 dans l'échantillon.

L'invention sera mieux comprise à l'aide de la description qui va suivre, donnée uniquement à titre d'exemple et faite en se référant aux dessins annexés, dans lesquels :

- la figure 1 est une vue schématique de l'installation pour déterminer l'âge d'une roche selon l'invention ;
- la figure 2 est une vue schématique du dispositif pour analyser un échantillon de roche selon l'invention ;
- la figure 3 est un diagramme représentant la méthode de détermination de l'âge de la roche à partir des mesures effectuées ;
- la figure 4 est un diagramme représentant le procédé d'analyse de la roche prélevée ; et
- la figure 5 est un diagramme représentant le procédé de réalisation de la base de données selon l'invention.

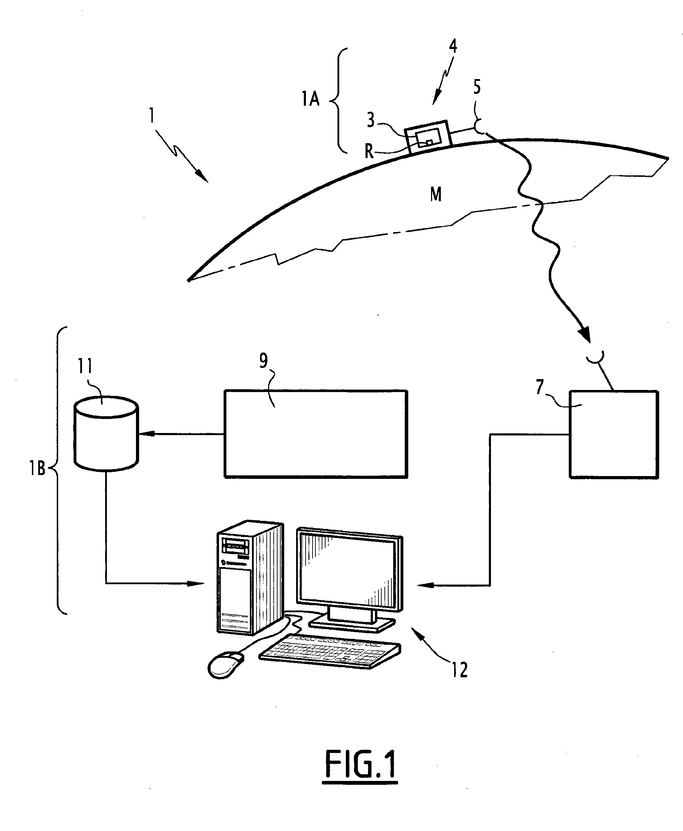

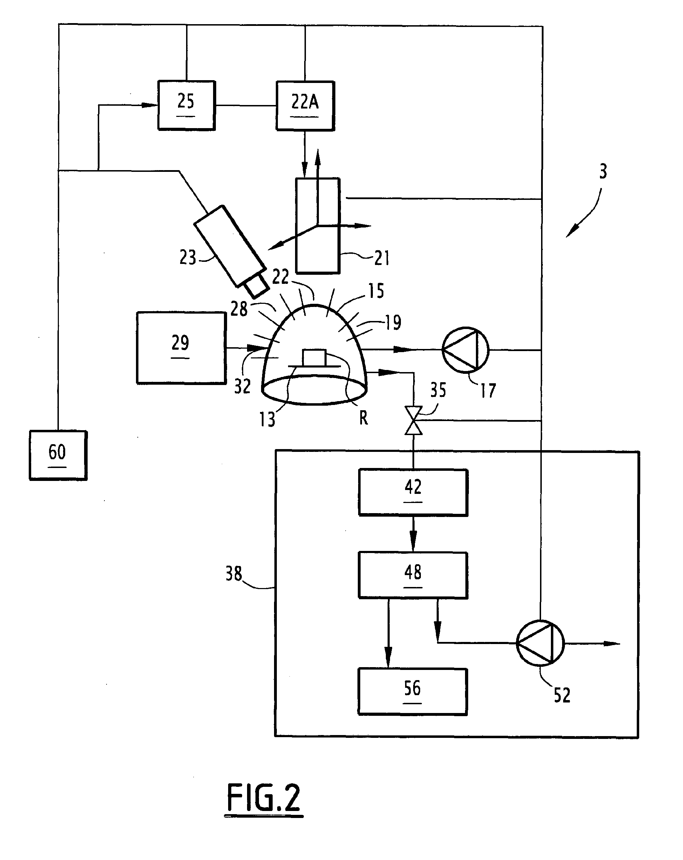

L'installation 1 schématisée sur la figure 1 a pour but la datation de roches R, notamment de roches magmatiques, situées dans des lieux inaccessibles à des opérateurs, tels que par exemple les fonds sous-marins ou des planètes du système solaire.
Elle est destinée notamment à la datation de roches de la planète Mars. A cet effet, l'installation comprend des moyens 1A situés sur la planète Mars, désignée par M et des moyens 1 B situés sur Terre qui coopèrent pour aboutir à la datation de la roche R.
Les moyens 1A présents sur Mars comportent un dispositif d'analyse 3 d'un échantillon E d'un minéral de la roche R. Ce dispositif d'analyse 3 se trouve sur le lieu de prélèvement de la roche R. Il est par exemple intégré à un robot 4, acheminé sur Mars par un lanceur spatial. Le robot 4 est équipé de moyens de télécommunication 5, qui permettent de transmettre les résultats des analyses effectuées au sein du dispositif d'analyse 3 à une station de réception et de transmission 7 située sur Terre. Les moyens de télécommunication 5 peuvent également recevoir des commandes effectuées par un opérateur sur Terre et transmises par l'intermédiaire de la station 7.
Un dispositif d'analyse 9, situé sur Terre, est destiné à analyser des échantillons de référence E1 de minéraux de roches de référence R1 prélevées sur Terre, en vue de la constitution d'une base de données 11. La station de réception 7 et la base de données 11 sont reliées à un ordinateur 12, propre à comparer les résultats des analyses effectuées sur Mars et reçus par la station de réception 7 avec les résultats d'analyses de référence contenus dans la base de données 11 pour calculer l'âge de la roche R prélevée sur Mars.
Le dispositif d'analyse 3 de l'échantillon E d'un minéral d'une roche R, représenté à la figure 2, comprend un porte-échantillon 13, placé dans une enceinte étanche 15. L'enceinte 15 est reliée à une pompe à vide 17. L'enceinte 15 comporte par ailleurs une fenêtre 19, qui permet d'introduire l'échantillon E dans l'enceinte 15 et de le placer sur le porte-échantillon 13 par l'intermédiaire d'un moyen de manipulation, par exemple un bras mécanique (non représenté).
Un laser 21 est destiné à traiter la roche R, confinée dans l'enceinte 15, au travers d'un hublot 22. Il est propre à ablater de celle-ci un échantillon E d'un minéral identifié et à former un plasma à partir de l'échantillon E ablaté. Le laser 21 émet de préférence dans l'ultraviolet lointain, c'est-à-dire à une longueur d'onde comprise entre 100 et 300 nm et de préférence d'environ 260 nm.
Un mécanisme 22A de positionnement relatif du laser 21 et du porte-échantillon 13 est propre à positionner le faisceau laser sur un point choisi de la roche R.
Une caméra 23, qui permet de visualiser la roche R placée sur le porte-échantillon 13, est reliée à une unité 25 de traitement de l'image provenant de la caméra 23.
La caméra 23 est placée, au sein du dispositif d'analyse 3, dans une configuration qui lui permet de viser la face de la roche R exposée vers le laser 21 au travers d'une fenêtre optique 28 formée dans l'enceinte 15.

Le dispositif d'analyse 3 comprend également un spectromètre ultraviolet 29, que l'on désignera par la suite par spectomètre UV 29, destiné à analyser le plasma issu de l'échantillon E lors du traitement au laser de la roche R. Le spectromètre UV 29 est placé, au sein du dispositif d'analyse 3, dans une configuration qui lui permet de viser l'intérieur de l'enceinte 15, par exemple par l'intermédiaire d'une fenêtre optique 32, formée dans l'enceinte 15.

L'enceinte 15 est également reliée, par exemple par l'intermédiaire d'une vanne 35, à un système d'analyse 38, propre à déterminer le nombre d'atomes d'argon 40 présents dans l'échantillon E. Le système d'analyse 38 comprend une pompe à sorption 42, propre à séparer les gaz actifs, issus du plasma formé par le laser 21 lors du traitement de l'échantillon E, des gaz inertes. La sortie de la pompe à sorption 42 est reliée à un dispositif de condensation 48, qui est lui-même relié à une pompe d'aspiration 52, ainsi qu'à un spectromètre de masse 56. Le spectromètre de masse 56 est destiné à analyser les gaz condensés issus du dispositif de condensation 48 pour déterminer leur composition isotopique et en déduire le nombre d'atomes d'argon 40, $^{40}Ar_{mesuré}$ dans l'échantillon E.

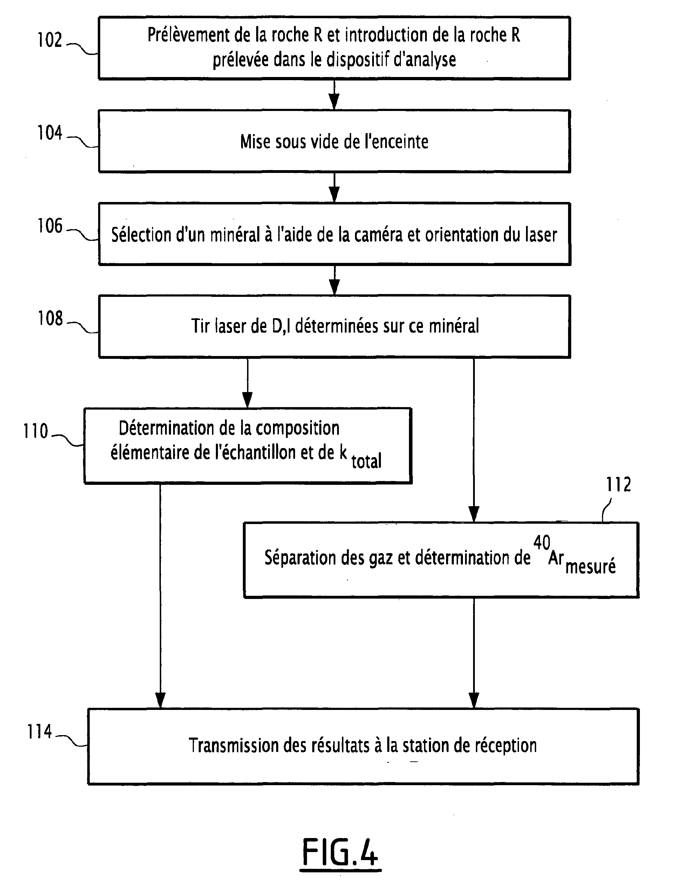

Une unité de commande 60 est reliée pour leur commande aux moyens de télécommunication 5, à la caméra 23, aux pompes 17 et 52, à la vanne 35, au mécanisme de positionnement 22A, au laser 21 et à l'unité de traitement 25. Cette unité de commande 60 est propre à mettre en oeuvre l'algorithme décrit ultérieurement en regard de la figure 4.

Le dispositif d'analyse 9 présent sur la Terre est propre à mettre en oeuvre les mêmes analyses que celles réalisées par le dispositif d'analyse 3 dans des conditions expérimentales comparables sur des roches de référence R1 de composition et d'âge connus. Les résultats obtenus à partir du dispositif d'analyse 9 sont stockés dans la base de données 11. Le dispositif d'analyse 9 comporte les mêmes éléments que le dispositif d'analyse 3, à l'exception du spectromètre UV 29. Le dispositif d'analyse 9 est moins miniaturisé que le dispositif d'analyse 3, puisqu'il n'est pas destiné à être embarqué dans un engin spatial. Le dispositif d'analyse 9 est manipulé directement par un opérateur et présente une automatisation moins importante que le dispositif d'analyse 3. Ainsi, il pourra ne pas comporter d'unité de commande 60, les commandes étant effectuées directement par un opérateur.

Les éléments constituant le dispositif 9, notamment le laser 21 et la pompe à vide 17, sont choisis pour que les conditions expérimentales soient identiques ou à tout le moins le plus proche possible de celles du dispositif d'analyse 3.

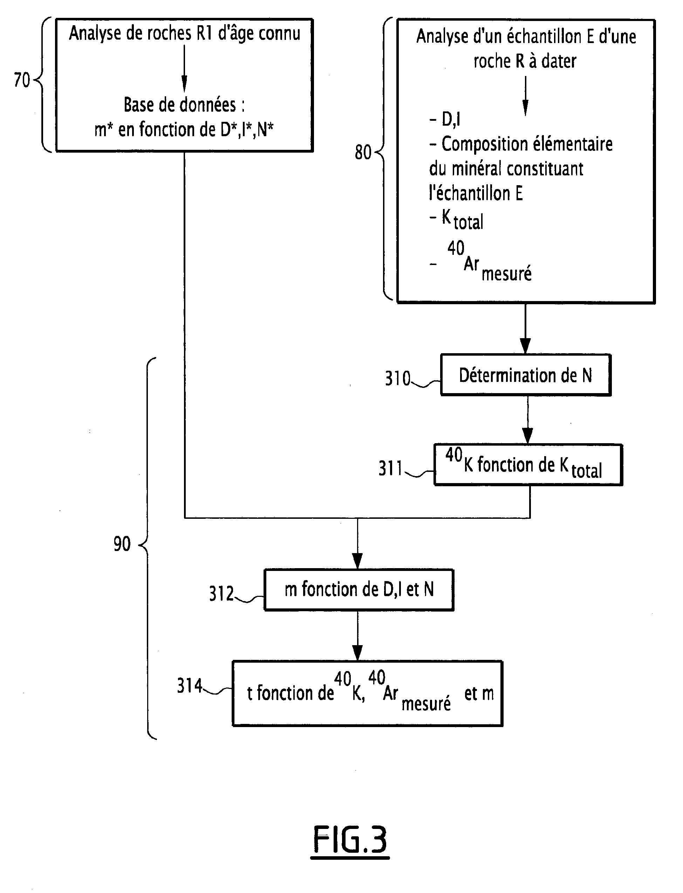

Comme illustré sur la figure 3, le procédé de datation comporte une phase préliminaire 70 de constitution d'une base de données 11 par mise en oeuvre du dispositif 9 sur des minéraux de roches de référence R1 de composition et d'âge connus, ainsi qu'une phase 80 d'acquisition des données d'essais sur des roches R à dater à partir du dispositif d'analyse 3 directement sur Mars.

Le procédé de datation comporte enfin une phase 90 de détermination de l'âge de la roche R à dater grâce aux résultats d'essais effectués sur la roche R à dater lors de la phase 80 et aux résultats d'analyses de référence effectuées à la phase 70 et stockés dans la base de données 11.

La phase d'analyse 80 depuis le dispositif d'analyse 3 de l'échantillon E d'un minéral de la roche R est détaillée sur la figure 4.

Dans une première étape 102, la roche R à dater est prélevée par l'intermédiaire du moyen de manipulation et est introduite dans l'enceinte 15 par la fenêtre 19. Elle est placée sur le porte échantillon 13.

A l'étape 104, l'unité de commande 60 actionne la pompe à vide 17 pour créer le vide dans l'enceinte 15.

A l'étape 106, la caméra 23 est activée par l'unité de commande 60 et acquiert des images de la roche R placée sur le porte échantillon 13. Ces images sont transmises par l'intermédiaire des moyens de télécommunication 5 à l'opérateur, qui sélectionne sur la base de ces images une zone de la roche R correspondant à un minéral identifié. Les coordonnées de ce minéral sont transmises à l'unité de commande 60, qui commande le déplacement du laser 21 par l'intermédiaire des moyens de positionnement 22A pour que celui-ci vise la zone de la roche R sélectionnée.

Lorsque le laser 21 a atteint la position souhaitée, un tir du laser 21 est effectué à l'étape 108 sous la commande de l'unité de commande 60. La durée D et l'intensité I du tir du laser 21 correspondent à ceux utilisés dans des essais de référence effectués sur des roches de référence R1 de nature identique stockés dans la base de données 11.

Le tir du laser 21 ablate un échantillon E du minéral sélectionné et le vaporise dans l'enceinte 15 sous la forme d'un plasma.

A l'étape 110, initiée dès que le plasma a été formé, le spectromètre UV 29, préalablement étalonné sur Terre, mesure à travers la fenêtre optique 32, le spectre d'émission du plasma et en déduit la composition élémentaire de l'échantillon E, dont la proportion en masse de potassium naturel, $K_{total}$ dans l'échantillon E ; la proportion en masse d'un élément étant définie comme la masse de cet élément par gramme d'échantillon E.

L'étape 112 est initiée dès que le plasma s'est recombiné et que les gaz issus du plasma se sont détendus dans l'enceinte 15. L'unité de commande 60 commande l'ouverture de la vanne 35, pour faire passer tous les gaz issus du plasma dans la pompe à sorption 42. Celle-ci sépare les gaz actifs des gaz inertes par adsorption des gaz actifs. Le dispositif de condensation 48 refroidit ensuite les gaz inertes, dont l'argon, à une température qui est de préférence inférieure à la température de l'azote liquide, c'est-à-dire à une température inférieure à environ 77,36 K, c'est-à-dire -195,79°C. A cette température, seul l'hélium, qui est indésirable, reste à l'état gazeux, tandis que les autres gaz sont condensés. A l'issue de la condensation, la pompe 52 est actionnée et élimine l'hélium. Ensuite, la vanne 35 est fermée et les gaz condensés sont détendus dans le spectromètre de masse 56. Le spectromètre de masse 56, préalablement étalonné sur Terre, analyse alors ce mélange gazeux et détermine le nombre d'atomes d'argon 40 présent dans l'échantillon E, $^{40}Ar_{mesuré}$.

A l'étape 114, l'unité de commande 60 centralise les résultats des analyses effectuées par le spectromètre UV 29, c'est-à-dire la composition élémentaire de l'échantillon E et la proportion en masse de potassium naturel, $K_{total}$ dans l'échantillon E, et les résultats des analyses effectuées par le spectromètre de masse 56, c'est-à-dire le nombre d'atomes d'argon 40 présent dans l'échantillon E, $^{40}Ar_{mesuré}$ et les transmet à la station 7 par l'intermédiaire des moyens de télécommunication 5.

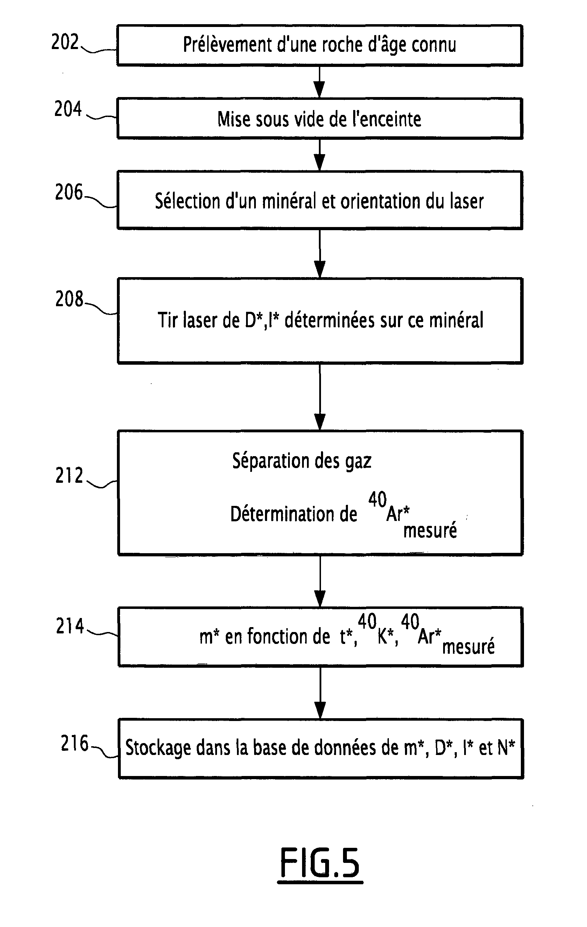

Pour constituer la base de données 11, le dispositif d'analyse 9 analyse sur Terre des échantillons de référence E1 de minéraux de roches de référence R1 d'âge connu selon un procédé illustré à la figure 5. Les roches de référence R1 d'âge connu sont prélevées sur la Terre et sont datées comme connu en soi par toute méthode adaptée. De plus, les différents minéraux des roches de référence R1 d'âge connu prélevées sont analysés par tout procédé connu adapté pour déterminer la proportion en masse de potassium 40 dans le minéral, $^{40}K^*$.

Les étapes 202, 204, 206 et 208 de ce procédé sont les mêmes que les étapes 102, 104, 106 et 108 du procédé mis en oeuvre par le dispositif d'analyse 3 et illustré à la figure 4. Cependant, le dispositif d'analyse 9 n'effectue pas l'étape 110 de détermination de la composition élémentaire de l'échantillon et de la proportion en masse de potassium naturel, $K_{total}$ dans l'échantillon, mais réalise directement l'étape 212 de séparation et d'analyse des gaz contenus dans le plasma du procédé illustré à la figure 4.

A l'issue de l'étape 212, le spectromètre de masse 56 a mesuré le nombre d'atomes d'argon 40 présent dans l'échantillon E, $^{40}Ar_{mesuré}{}^*$.

Un opérateur ou des moyens de télécommunication appropriés transfèrent ensuite à l'ordinateur 12 :

- $^{40}Ar_{mesuré}{}^*$
- la nature N* du minéral identifié préalablement par toute méthode connue adaptée, sur lequel le tir du laser 21 a été effectué à l'étape 208
- les caractéristiques I*, D* du tir du laser 21 effectué à l'étape 208
- l'âge t* de la roche de référence R1 prélevée, déterminé préalablement par toute méthode connue adaptée.

A l'étape 214, l'ordinateur 12 calcule la masse m* de l'échantillon de référence E1 en utilisant la formule suivante :

$$m^* = \frac{^{40}Ar_{mesuré}{}^*}{^{40}Ar^*}$$

où

$$Ar^* = \left(\frac{^{40}K^* \times N_A}{M}\right) \times \frac{\lambda_{Ar}}{\lambda_{Ar} + \lambda_{Ca}} \times \left(e^{t^* \times (\lambda_{Ar} + \lambda_{Ca})} - 1\right),$$

Avec

- m* est la masse de l'échantillon de référence E1,
- $^{40}Ar_{mesuré}{}^*$ est le nombre d'atomes d'argon 40 mesurés dans l'échantillon de référence E1 à l'étape 212,
- $^{40}Ar^*$ est le nombre d'atomes d'argon par gramme d'échantillon de référence E1,
- $^{40}K^*$ est la proportion en masse de potassium 40 dans le minéral, déterminée par toute méthode connue adaptée,
- $N_A$ est la constante d'Avogadro
- M est la masse molaire du potassium 40
- $\lambda_{Ar}$ et $\lambda_{Ca}$ sont respectivement les constantes de désintégration correspondant à la formation d'argon 40 radiogénique et à la formation de calcium 40 radiogénique à partir du potassium 40 et
- t* est l'âge de la roche de référence R1, calculé par toute méthode connue adaptée.

Enfin, à l'étape 216, l'ordinateur 12 stocke dans la base de données 11 la masse m* de l'échantillon de référence E1 qu'il a calculée dans l'étape 214, associée à la nature N* du minéral constituant l'échantillon de référence E1 et aux caractéristiques (durée D*, intensité I*) du tir du laser 21 effectué à l'étape 206 du procédé de constitution de la base de données 11.

Les étapes 202, 204, 206, 208, 212, 214 et 216 sont répétées pour des minéraux de natures N* différentes et/ou pour différentes caractéristiques de tir (D*, I*).

La figure 3 détaille le procédé de détermination de l'âge de la roche R par l'ordinateur 12 à partir de la base de données 11, constituée lors de la phase 70 et des résultats des analyses réalisées par le spectromètre de masse 56 et par le spectromètre UV 29 sur l'échantillon E de la roche R lors de la phase 80.

A l'étape 310, l'ordinateur 12 déduit de la composition élémentaire de l'échantillon E déterminée par le spectromètre UV 29 à l'étape 110, la nature N du minéral constituant l'échantillon E.

A l'étape 311, l'ordinateur 12 effectue le calcul suivant pour déterminer la proportion en masse de potassium 40, $^{40}K$, dans l'échantillon E :

$$^{40}K = K_{total} \times F \,,$$

où

- $K_{total}$ est la proportion en masse de potassium naturel dans l'échantillon E,
- F est la fraction, connue, de potassium 40 parmi le potassium naturel.

L'ordinateur 12 recherche ensuite à l'étape 312 dans la base de données 11 un échantillon de référence E1 de même nature N* que l'échantillon E, obtenu avec les mêmes caractéristiques D*, I* du tir de laser 21, que l'échantillon E. Il prend alors la masse m* correspondante et la fait correspondre à la masse m de l'échantillon E.

Enfin, l'ordinateur 12 applique, à l'étape 314, la formule suivante pour calculer l'âge de l'échantillon E de roche

$$t = \frac{1}{\lambda_{Ar} + \lambda_{Ca}} \times \ln(\frac{\lambda_{Ar} + \lambda_{Ca}}{\lambda_{Ar}} \times \frac{\frac{^{40}Ar_{mesuré}}{m}}{\frac{^{40}K \times N_A}{M}} + 1) \,,$$

où

- t est l'âge de la roche R prélevée sur Mars,
- $\lambda_{Ar}$ et $\lambda_{Ca}$ sont respectivement les constantes de désintégration correspondant à la formation d'argon 40 radiogénique et à la formation de calcium 40 radiogénique à partir du potassium 40,
- $^{40}Ar_{mesuré}$ est le nombre d'atomes d'argon 40 mesuré dans l'échantillon,
- m est la masse de l'échantillon calculée par l'ordinateur 12,
- $^{40}K$ est la proportion en masse de potassium 40 dans l'échantillon.
- $N_A$ est la constante d'Avogadro
- M est la masse molaire du potassium 40

La présente invention présente l'avantage de permettre une évaluation précise de la masse m de l'échantillon ablaté par le laser 21, puisque cette évaluation est faite à partir de la base de données 11 constituée sur Terre dans les mêmes conditions expérimentales que sur Mars. Une telle évaluation de la masse est utile, car la méthode de traitement de l'échantillon, qui fait appel au laser 21 ne permet pas de mesure directe sur Mars de la masse ablatée par le laser.

Le spectromètre 29 est, de manière plus générale, un spectrophotomètre.

Dans tout ce qui précède, on entend par minéral un minéral unique ou un ensemble de minéraux. Ainsi, selon les dimensions du faisceau laser, et en fonction des tailles des minéraux de la roche, l'échantillon ablaté comprend un minéral unique ou un ensemble de minéraux, éventuellement de natures différentes. Dans le cas où l'on a tiré sur un ensemble de minéraux de la roche, la nature du minéral constituant l'échantillon correspond à la nature de cet ensemble de minéraux, déterminée à partir de la composition élémentaire de l'échantillon.

**Revendications**

1. Installation (1) de détermination de l'âge d'un échantillon (E) d'un minéral d'une roche (R), l'installation (1) comprenant

   - un dispositif (3) pour analyser un échantillon (E) d'un minéral de la roche (R) comprenant un nombre ($^{40}Ar_{mesuré}$) d'atomes d'argon 40 et une proportion en masse ($^{40}K$) de potassium 40 dans l'échantillon (E), le dispositif (3) d'analyse comportant

      - des moyens de traitement (21) de la roche (R) destinés à extraire un échantillon (E) d'un minéral de la roche (R) et à libérer l'argon 40 et le potassium naturel, qui comprend le potassium 40, de l'échantillon (E) ;
      - des premiers moyens d'analyse (56) destinés à déterminer le nombre ($^{40}Ar_{mesuré}$) d'atomes d'argon 40 dans l'échantillon (E) ; et
      - des seconds moyens d'analyse (12, 29) destinés à déterminer la proportion en masse ($^{40}K$) de potassium 40 dans l'échantillon (E) ;
      **caractérisé en ce que**
      - les moyens de traitement de la roche (R) comportent un laser (21) destiné à tirer sur la roche (R) pour ablater l'échantillon (E) de la roche (R) et former un plasma à partir de l'échantillon (E) ;
      - les premiers moyens d'analyse (56) sont propres à analyser des gaz issus du plasma pour déterminer le nombre ($^{40}Ar_{mesuré}$) d'atomes d'argon 40 dans l'échantillon (E) ;
      - les seconds moyens d'analyse (12, 29) sont propres à analyser le plasma formé par le laser (21) pour déterminer la proportion en masse ($^{40}K$) de potassium 40 dans l'échantillon (E) ;

      et **caractérisée en ce que** l'installation (1) comprend en outre
      - des moyens (9, 12) destinés à constituer une base de données (11) à partir d'au moins une roche de référence (R1) d'age (t\*) connu, la base de données (11) associant la masse (m\*) d'un échantillon de référence (E1) d'un minéral de la roche de référence (R1) aux caractéristiques (D\*, I\*) du tir du laser (21) et à la nature (N\*) du minéral constituant l'échantillon de référence (E1) ;
      - des moyens (12) destinés à déterminer, à l'aide de la base de données (11) ainsi constituée, la masse (m) de l'échantillon (E) à partir des caractéristiques (D, I) du tir laser effectué pour ablater l'échantillon (E) et de la nature (N) du minéral constituant l'échantillon (E) ; et
      - des moyens (12) destinés à calculer l'âge (t) de la roche (R) à partir de la masse (m) de l'échantillon (E), du nombre ($^{40}Ar_{mesuré}$) d'atomes d'argon 40 et de la proportion en masse ($^{40}K$) de potassium 40 dans l'échantillon (E).

2. Installation (1) selon la revendication 1, dans laquelle les moyens (9, 12) destinés à constituer la base de données (11) à partir d'au moins une roche de référence (R1) d'âge (t\*) connu, comportant un nombre ($^{40}Ar_{mesuré}$\*) d'atomes d'argon 40 sont propres à :

   - traiter (208) la au moins une roche de référence (R1) à l'aide des moyens de traitement (21) de manière à extraire un échantillon de référence (E1) d'un minéral de la au moins une roche de référence (R1) et à libérer l'argon 40 contenu dans l'échantillon de référence (E1), l'étape de traitement consistant à effectuer (208) un tir sur la au moins une roche de référence (R1) avec le laser (21) pour ablater un échantillon de référence (E1) d'un minéral de la au moins une roche de référence (R1) et pour former un plasma à partir de l'échantillon de référence (E1) ;
   - déterminer (212) le nombre ($^{40}Ar_{mesuré}$\*) d'atomes d'argon 40 dans les gaz issus du plasma formé à partir de l'échantillon de référence (E1) à l'aide des moyens d'analyse (56) ;
   - calculer (214) la masse (m\*) de l'échantillon de référence (E1) à partir de l'âge (t\*) de la au moins une roche de référence (R1) dont il est issu, de la nature (N\*) du minéral qui constitue l'échantillon de référence (E1) et du nombre ($^{40}Ar_{mesuré}$\*) d'atomes d'argon 40 dans l'échantillon de référence (E1) ;
   - associer (216) la masse (m\*) de l'échantillon de référence (E1) aux caractéristiques (D\*, I\*) du tir effectué par le laser (21) et à la nature (N\*) du minéral qui constitue l'échantillon de référence (E1).

3. Installation (1) selon l'une quelconque des revendications précédentes, dans laquelle les premiers moyens d'analyse du dispositif d'analyse (3) comprennent un spectromètre de masse (56).

4. Installation (1) selon l'une quelconque des revendications précédentes, dans laquelle les seconds moyens d'analyse du dispositif d'analyse (3) comprennent un spectromètre ultraviolet (29).

**5.** Installation (1) selon l'une quelconque des revendications précédentes, dans laquelle le laser (21) est destiné à émettre dans l'ultraviolet lointain.

**6.** Installation (1) selon l'une quelconque des revendications précédentes, dans laquelle le dispositif d'analyse (3) comporte en outre des moyens de visualisation (23) de la roche (R), ainsi que des moyens de guidage (22A) du laser (21) pour un tir sur une zone choisie de la roche (R).

**7.** Installation (1) selon l'une des revendications précédentes, dans laquelle le dispositif d'analyse (3) comporte en outre une enceinte (15) destinée à accueillir la roche (R) et à être mise sous vide, ainsi qu'un moyen de mise sous vide (17) de l'enceinte (15).

**8.** Installation (1) selon l'une des revendications précédentes, dans laquelle le dispositif d'analyse (3) comporte en outre des moyens de séparation (42, 48, 52) des gaz issus du plasma.

**9.** Installation (1) selon l'une des revendications précédentes, dans laquelle les moyens de séparation (42, 48, 52) des gaz issus du plasma comportent une pompe à sorption (42), ainsi qu'un dispositif de condensation des gaz (48).

**10.** Procédé de détermination de l'âge d'un échantillon (E) d'un minéral d'une roche (R), comportant les étapes consistant à :

- commander la détermination (80) du nombre ($^{40}Ar_{mesuré}$) d'atomes d'argon 40 et de la proportion en masse ($^{40}K$) de potassium 40 dans l'échantillon (E) en

- commandant (108) le traitement de la roche (R) à l'aide de moyens de traitement (21) de manière à extraire un échantillon (E) d'un minéral de la roche (R) et à libérer l'argon 40 et le potassium naturel, qui comprend le potassium 40 de l'échantillon (E) ;
- commandant la détermination (112) du nombre ($^{40}Ar_{mesuré}$) d'atomes d'argon 40 dans l'échantillon (E) à l'aide de premiers moyens d'analyse (56) ;
- commandant la détermination (110) de la proportion en masse ($^{40}K$) de potassium 40 dans l'échantillon (E) à l'aide de seconds moyens d'analyse (12, 29) ;
**caractérisé en ce que**
- l'étape de traitement de la roche (R) consiste à effectuer un tir sur la roche (R) avec un laser (21) pour ablater un échantillon (E) d'un minéral de la roche (R) et pour former un plasma à partir de l'échantillon (E) ;
- on détermine le nombre ($^{40}Ar_{mesuré}$) d'atomes d'argon 40 dans les gaz issus du plasma formé à partir de l'échantillon (E) à l'aide des premiers moyens d'analyse (56) ;
- on détermine (110) de la proportion en masse ($^{40}K$) de potassium 40 dans le plasma formé à partir de l'échantillon (E) à l'aide des seconds moyens d'analyse (12, 29) ;

- on réalise (70) une base de données (11) associant la masse (m*) d'un échantillon de référence (E1) d'un minéral d'une roche de référence (R1) aux caractéristiques (D*, I*) du tir du laser (21) et à la nature (N*) du minéral constituant l'échantillon de référence (E1) ;
- on commande la détermination (312), à l'aide de la base de données (11) ainsi constituée, de la masse (m) de l'échantillon (E) à partir des caractéristiques (D, I) du tir laser effectué pour ablater l'échantillon (E) et de la nature (N) du minéral constituant l'échantillon (E) ;
- on commande le calcul (314) de l'âge (t) de la roche (R) à l'aide de la masse (m) de l'échantillon (E), du nombre ($^{40}Ar_{mesuré}$) d'atomes d'argon 40 et de la proportion en masse ($^{40}K$) de potassium 40 dans l'échantillon (E) ;

les étapes de commande étant réalisées au moyen d'une unité de commande (60).

**11.** Procédé de détermination de l'âge d'un échantillon (E) d'un minéral d'une roche (R) selon la revendication 10, dans lequel l'étape de réalisation de la base de données (11) comprend les étapes de :

- traiter (208) la au moins une roche de référence (R1) à l'aide de moyens de traitement (21) de manière à extraire un échantillon de référence (E1) d'un minéral de la au moins une roche de référence (R1) et à libérer l'argon 40 contenu dans l'échantillon de référence (E1), l'étape de traitement consistant à effectuer (208) un tir sur la au moins une roche de référence (R1) avec un laser (21) pour ablater un échantillon de référence (E1) d'un minéral de la au moins une roche de référence (R1) et pour former un plasma à partir de l'échantillon (E1) ; ;
- déterminer (212) le nombre ($^{40}Ar_{mesuré}$*) d'atomes d'argon 40 dans les gaz issus du plasma formé à partir de l'échantillon de référence (E1) à l'aide de moyens d'analyse (56) ;
- calculer (214) la masse (m*) de l'échantillon de référence (E1) à partir de l'âge (t*) de la au moins une roche de référence (R1) dont il est issu, de la nature (N*) du minéral qui constitue l'échantillon de référence (E1) et du nombre ($^{40}Ar_{mesuré}$*) d'atomes d'argon 40 dans l'échantillon de référence (E1) ;
- associer (216) la masse (m*) de l'échantillon de référence (E1) aux caractéristiques (D*, I*) du tir effectué par le laser (21) et à la nature (N*) du minéral qui constitue l'échantillon de référence (E1).

**Patentansprüche**

1.  Anlage (1) zur Bestimmung des Alters einer Probe (E) eines Minerals eines Gesteins (R), wobei die Anlage (1) umfasst

    - eine Vorrichtung (3) zum Analysieren einer Probe (E) eines Minerals des Gesteins (R), das eine Anzahl ($^{40}Ar_{gemessen}$) von Argon 40 Atomen und einen Massenanteil ($^{40}K$) von Kalium 40 in der Probe (E) umfasst, wobei die Analysevorrichtung aufweist

    - Mittel (21) zur Behandlung des Gesteins, die vorgesehen sind, eine Probe (E) eines Minerals des Gesteins (R) zu extrahieren und das Argon 40 und das natürliche Kalium, das das Kalium 40 umfasst, von der Probe (E) freizusetzen;
    - erste Analysemittel (56), die vorgesehen sind, die Anzahl ($^{40}Ar_{gemessen}$) an Argon 40 Atomen in der Probe (E) zu bestimmen; und
    - zweite Analysemittel (12, 29), die vorgesehen sind, den Massenanteil ($^{40}K$) an Kalium 40 in der Probe (E) zu bestimmen;
    **dadurch gekennzeichnet, dass**
    - die Mittel zur Behandlung des Gesteins (R) einen Laser (21) aufweisen, der vorgesehen ist, auf das Gestein (R) zu schießen, um die Probe (E) von dem Gestein (R) abzutragen und ein Plasma aus der Probe (E) zu bilden;
    - die ersten Analysemittel (56) geeignet sind, Gase aus dem Plasma zu analysieren, um die Anzahl ($^{40}Ar_{gemessen}$) an Argon 40 Atomen in der Probe (E) zu bestimmen;
    - die zweiten Analysemittel (12, 29) geeignet sind, das von dem Laser (21) gebildete Plasma zu analysieren, um den Massenanteil ($^{40}K$) an Kalium 40 in der Probe (E) zu bestimmen;

    und **dadurch gekennzeichnet, dass** die Anlage (1) außerdem umfasst
    - Mittel (9, 12), die vorgesehen sind, eine Datenbank (11) aus mindestens einem Referenzgestein (R1) von bekanntem Alter (t*) zu bilden, wobei die Datenbank (11) die Masse (m*) einer Referenzprobe (E1) eines Minerals des Referenzgesteins (R1) den charakteristischen Merkmalen (D*, I*) des Laserschusses (21) und der Natur (N*) des Minerals, das die Referenzprobe (E1) bildet, zuordnet;
    - Mittel (12), die vorgesehen sind, mit Hilfe der so gebildeten Datenbank (11) die Masse (m) der Probe (E) aus den charakteristischen Merkmalen (D, I) des Laserschusses, der zum Abtragen der Probe (E) ausgeführt wurde, und aus der Natur (N) des die Probe (E) bildenden Minerals zu bestimmen; und
    - Mittel (12), die vorgesehen sind, das Alter (t) des Gesteins (R) aus der Masse (m) der Probe (E), der Anzahl ($^{40}Ar_{gemessen}$) an Argon 40 Atomen und dem Massenanteil ($^{40}K$) an Kalium 40 in der Probe (E) zu berechnen.

2.  Anlage (1) nach Anspruch 1, bei der die Mittel (9, 12), die vorgesehen sind, die Datenbank (11) aus mindestens einem Referenzgestein (R1) eines bekannten Alters (t*) zu bilden, das eine Anzahl ($^{40}Ar_{gemessen}$*) an Argon 40 Atomen aufweist, geeignet sind:

    - das mindestens eine Referenzgestein (R1) mithilfe der Mittel zum Behandeln (21) derart zu behandeln (208), dass eine Referenzprobe (E1) eines Minerals des mindestens einen Referenzgesteins (R1) extrahiert wird und in der Referenzprobe (E1) enthaltenes Argon 40 freigesetzt wird, wobei der Behandlungsschritt darin besteht, einen Schuss mit dem Laser (21) auf das mindestens eine Referenzgestein (R1) auszuführen (208), um eine Referenzprobe (E1) eines Minerals des mindestens einen Referenzgesteins (R1) abzutragen und ein Plasma aus der Referenzprobe (E1) zu bilden;
    - die Anzahl ($^{40}Ar_{gemessen}$*) an Argon 40 Atomen in den Gasen aus dem Plasma, das aus der Referenzprobe (E1) gebildet wurde, mithilfe der Analysemittel (56) zu bestimmen (212);
    - die Masse (m*) der Referenzprobe (E1) aus dem Alter (t*) des mindestens einen Referenzgesteins (R1), aus dem sie herrührt, aus der Natur (N*) des Minerals, das die Referenzprobe (E1 bildet, und der Anzahl ($^{40}Ar_{gemessen}$*) an Argon 40 Atomen in der Referenzprobe (E1) zu berechnen (214);

- die Masse (m*) der Referenzprobe (E1) den charakteristischen Merkmalen (D*, I*) des von dem Laser (21) ausgeführten Schusses und der Natur (N*) des Minerals, das die Referenzprobe (E1) bildet, zuzuordnen (216).

3. Anlage (1) nach einem beliebigen der vorhergehenden Ansprüche, bei der die ersten Analysemittel der Analysevorrichtung (3) ein Massenspektrometer (56) aufweisen.

4. Anlage (1) nach einem beliebigen der vorhergehenden Ansprüche, bei der die zweiten Analysemittel der Analysevorrichtung (3) ein Ultraviolett-Spektrometer (29) aufweisen.

5. Anlage (1) nach einem beliebigen der vorhergehenden Ansprüche, bei der der Laser (21) vorgesehen ist, fernes Ultraviolett abzustrahlen.

6. Anlage (1) nach einem beliebigen der vorhergehenden Ansprüche, bei der die Analysevorrichtung (3) außerdem Mittel zur Visualisierung (23) des Gesteins (R) sowie Mittel zur Führung (22A) des Lasers (21) für einen Schuss auf eine ausgewählte Zone des Gesteins (R) aufweist.

7. Anlage (1) nach einem der vorhergehenden Ansprüche, bei der die Analysevorrichtung (3) außerdem eine Umfassung (15), die vorgesehen ist, das Gestein (R) aufzunehmen und unter Vakuum gesetzt zu werden, sowie ein Mittel (17) zur Erzeugung von Vakuum in der Umfassung (15) aufweist.

8. Anlage (1) nach einem der vorhergehenden Ansprüche, bei der die Analysevorrichtung (3) außerdem Mittel zur Trennung (42, 48, 52) der aus dem Plasma stammenden Gase aufweist.

9. Anlage (1) nach einem der vorhergehenden Ansprüche, bei der die Mittel zur Trennung (42, 48, 52) der aus dem Plasma stammenden Gase eine Sorptionspumpe (42) sowie eine Vorrichtung zur Kondensation der Gase (48) aufweisen.

10. Verfahren zur Bestimmung des Alters einer Probe (E) eines Minerals eines Gesteins (R), das die Schritt umfasst, die darin bestehen:

- die Bestimmung (80) der Anzahl ($^{40}Ar_{gemessen}$) an Argon 40 Atomen und des Massenanteils ($^{40}K$) an Kalium 40 in der Probe (E) zu steuern, indem

- die Behandlung des Gesteins (R) mit Hilfe von Behandlungsmitteln (21) gesteuert wird (108), um eine Probe (E) eines Minerals des Gesteins (R) zu extrahieren und das Argon 40 und das natürliche Kalium, das das Kalium 40 umfasst, aus der Probe (E) freizusetzen;
- die Bestimmung (112) der Anzahl ($^{40}Ar_{gemessen}$) an Argon 40 Atomen in der Probe (E) mithilfe der ersten Analysemittel (56) zu steuern;
- die Bestimmung (110) des Massenanteils ($^{40}K$) des Kalium 40 in der Probe (E) mit Hilfe der zweiten Analysemittel (12, 29) zu steuern;
**dadurch gekennzeichnet, dass**
- der Schritt der Behandlung des Gesteins (R) darin besteht, einen Schuss mit einem Laser (21) auf das Gestein (R) auszuführen, um eine Probe (E) eines Minerals des Gesteins (R) abzutragen und ein Plasma aus der Probe (E) zu bilden;
- die Anzahl ($^{40}Ar_{gemessen}$) an Argon 40 Atomen in den aus dem Plasma stammenden Gasen, das aus der Probe (E) gebildet wurde, mit Hilfe der ersten Analysemittel (56) bestimmt wird;
- der Massenanteil ($^{40}K$) an Kalium 40 in dem aus der Probe (E) gebildeten Plasma mit Hilfe der zweiten Analysemittel (12, 29) bestimmt wird (110);

- eine Datenbank (11) hergestellt wird (70), die die Masse (m*) einer Referenzprobe (E1) eines Minerals eines Referenzgesteins (R1) den charakteristischen Merkmalen (D*, I*) des Laserschusses (21) und der Natur (N*) des die Referenzprobe bildenden Minerals zuordnet;
- die Bestimmung (312) der Masse (m) der Probe (E) mit Hilfe der so gebildeten Datenbank (11) aus den charakteristischen Merkmalen (D, I) des Laserschusses, der zum Abtragen der Probe (E) ausgeführt wurde, und der Natur (N) des die Probe (E) bildenden Minerals gesteuert wird;
- die Berechnung (314) des Alters (t) des Gesteins (R) mit Hilfe der Masse (m) der Probe (E), der Anzahl ($^{40}Ar_{gemessen}$) an Argon 40 Atomen und dem Massenanteil ($^{40}K$) an Kalium 40 in der Probe (E) gesteuert wird;

wobei die Schritte des Steuerns mittels einer Steuereinheit (60) durchgeführt werden.

11. Verfahren zur Bestimmung des Alters einer Probe (E) eines Minerals eines Gesteins (R) nach Anspruch 10, bei dem der Schritt der Herstellung der Datenbank (11) die folgenden Schritte umfasst:

- Behandeln (208) des mindestens einen Referenzgesteins (R1) mit Hilfe von Behandlungsmitteln (21), derart, dass eine Referenzprobe (E1) eines Minerals des Referenzgesteins (R1) extrahiert wird und in der Referenzprobe (E1) enthaltenes Argon (40) freigesetzt wird, wobei der Behandlungsschritt darin besteht, einen Schuss mit einem Laser (21) auf das mindestens eine Referenzgestein (R) auszuführen, um eine Referenzprobe (E1) eines Minerals des mindestens einen Referenzgesteins (R1) abzutragen und ein Plasma aus der Probe (E1) zu bilden;
- Bestimmen (212) der Anzahl ($^{40}Ar_{gemessen}$*) an Argon 40 Atomen in den aus dem Plasma stammenden Gasen, das aus der Referenzprobe (E1) gebildet wurde, mit Hilfe der Analysemittel (56);
- Berechnen (214) der Masse (m*) der Referenzprobe (E1) aus dem Alter (t*) des mindestens einen Referenzgesteins (R1), aus dem sie herrührt, der Natur (N*) des Minerals, das die Referenzprobe (E1 bildet, und der Anzahl ($^{40}Ar_{gemessen}$*) an Argon 40 Atomen in der Referenzprobe (E1);
- Zuordnen (216) der Masse (m*) der Referenzprobe (E1) zu den charakteristischen Merkmalen (D*, I*) des von dem Laser (21) ausgeführten Schusses und zu der Natur (N*) des Minerals, das die Referenzprobe (E1) bildet.

## Claims

1. Installation (1) for determining the age of a sample (E) of a mineral of a rock (R), the installation (1) comprising:

- a device (3) for analysing a sample (E) of a mineral of the rock (R) comprising a number ($^{40}Ar_{measured}$) of argon-40 atoms and a proportion by mass ($^{40}K$) of potassium-40 in the sample (E), the analysis device (3) comprising
- means (21) for treating the rock (R) which are to extract a sample (E) of a mineral of the rock (R) and free the argon-40 and the natural potassium, which comprises potassium-40, from the sample (E);
- first analysis means (56) which are to determine the number ($^{40}Ar_{measured}$) of argon-40 atoms in the sample (E); and
- second analysis means (12, 29) which are to determine the proportion by mass ($^{40}K$) of potassium-40 in the sample (E);
**characterised in that**
- the means for treating the rock (R) comprise a laser (21) which is to be fired at the rock (R) in order to ablate the sample (E) of the rock (R) and form a plasma from the sample (E);
- the first analysis means (56) are capable of analysing the gases obtained from the plasma in order to determine the number ($^{40}Ar_{measured}$) of argon-40 atoms in the sample (E);
- the second analysis means (12, 29) are capable of analysing the plasma formed by the laser (21) in order to determine the proportion by mass ($^{40}K$) of potassium-40 in the sample (E);
and **characterised in that** the installation (1) further comprises
- means (9, 12) which are to establish a database (11) from at least one reference rock (R1) of known age (t*), the database (11) associating the mass (m*) of a reference sample (E1) of a mineral of the reference rock (R1) with the characteristics (D*, I*) of fire of the laser (21) and with the nature (N*) of the mineral constituting the reference sample (E1);
- means (12) which are to determine, with the aid of the database (11) so established, the mass (m) of the sample (E) from the characteristics (D, I) of the laser fire effected in order to ablate the sample (E) and from the nature (N) of the mineral constituting the sample (E); and
- means (12) which are to calculate the age (t) of the rock (R) from the mass (m) of the sample (E), from the number ($^{40}Ar_{measured}$) of argon-40 atoms and from the proportion by mass ($^{40}K$) of potassium-40 in the sample (E).

2. Installation (1) according to claim 1, wherein the means (9, 12) which are to establish the database (11) from at least one reference rock (R1) of known age (t*), comprising a number ($^{40}Ar_{measured}$*) of argon-40 atoms, are capable of:

- treating (208) the at least one reference rock (R1) with the aid of the treatment means (21) so as to extract a reference sample (E1) of a mineral of the at least one reference rock (R1 and free the argon-40 contained in the reference sample (E1), the treatment step consisting in firing (208) at the at least one reference rock (R1) with the laser (21) in order to ablate a reference sample (E1) of a mineral of the at least one reference rock (R1) and form a plasma from the reference sample (E1);

- determining (212) the number ($^{40}Ar_{measured}$*) of argon-40 atoms in the gases obtained from the plasma formed from the reference sample (E1) with the aid of the analysis means (56);
- calculating (214) the mass (m*) of the reference sample (E1) from the age (t*) of the at least one reference rock (R1) from which it is obtained, from the nature (N*) of the mineral constituting the reference sample (E1) and from the number ($^{Ar}40_{measured}$*) of argon-40 atoms in the reference sample (E1);
- associating (216) the mass (m*) of the reference sample (E1) with the characteristics (D*, I*) of the fire effected by the laser (21) and with the nature (N*) of the mineral constituting the reference sample (E1).

3. Installation (1) according to any one of the preceding claims, wherein the first analysis means of the analysis device (3) comprise a mass spectrometer (56).

4. Installation (1) according to any one of the preceding claims, wherein the second analysis means of the analysis device (3) comprise an ultraviolet spectrometer (29).

5. Installation (1) according to any one of the preceding claims, wherein the laser (21) is to emit in the far ultraviolet.

6. Installation (1) according to any one of the preceding claims, wherein the analysis device (3) further comprises means (23) for visualisation of the rock (R) as well as means (22A) for guiding the laser (21) to fire at a chosen zone of the rock (R).

7. Installation (1) according to any one of the preceding claims, wherein the analysis device (3) further comprises an enclosure (15) which is to receive the rock (R) and be placed under vacuum, as well as a means (17) for placing the enclosure (15) under vacuum.

8. Installation (1) according to any one of the preceding claims, wherein the analysis device (3) further comprises means (42, 48, 52) for separating the gases obtained from the plasma.

9. Installation (1) according to any one of the preceding claims, wherein the means (42, 48, 52) for separating the gases obtained from the plasma comprise a sorption pump (42) as well as a device for condensation of the gases (48).

10. Method for determining the age of a sample (E) of a mineral of a rock (R), comprising steps consisting in:

- commanding the determination (80) of the number ($^{40}Ar_{measured}$) of argon-40 atoms and of the proportion by mass ($^{40}K$) of potassium-40 in the sample (E) by

- commanding (108) the treatment of the rock (R) with the aid of treatment means (21) so as to extract a sample (E) of a mineral of the rock (R) and free the argon-40 and the natural potassium, which comprises potassium-40, from the sample (E);
- commanding the determination (112) of the number ($^{40}Ar_{measured}$) of argon-40 atoms in the sample (E) with the aid of first analysis means (56);
- commanding the determination (110) of the proportion by mass ($^{40}K$) of potassium-40 in the sample (E) with the aid of second analysis means (12, 29);
**characterised in that**
- the step of treating the rock (R) consists in firing at the rock (R) with a laser (21) in order to ablate a sample (E) of a mineral of the rock (R) and form a plasma from the sample (E);
- the number ($^{40}Ar_{measured}$) of argon-40 atoms in the gases obtained from the plasma formed from the sample (E) is determined with the aid of the first analysis means (56);
- the proportion by mass ($^{40}K$) of potassium-40 in the plasma formed from the sample (E) is determined (110) with the aid of the second analysis means (12, 29);

- there is produced (70) a database (11) which associates the mass (m*) of a reference sample (E1) of a mineral of a reference rock (R1) with the characteristics (D*, I*) of fire of the laser (21) and with the nature (N*) of the mineral constituting the reference sample (E1);
- there is commanded the determination (312), with the aid of the database (11) so established, of the mass (m) of the sample (E) from the characteristics (D, I) of the laser fire effected in order to ablate the sample (E) and from the nature (N) of the mineral constituting the sample (E);

- there is commanded the calculation (314) of the age (t) of the rock (R) with the aid of the mass (m) of the sample (E), the number ($^{40}Ar_{measured}$) of argon-40 atoms and the proportion by mass ($^{40}K$) of potassium-40 in the sample (E);

the command steps being carried out by means of a control unit (60).

11. Method for determining the age of a sample (E) of a mineral of a rock (R) according to claim 10, wherein the step of producing the database (11) comprises the steps of:

- treating (208) the at least one reference rock (R1) with the aid of treatment means (21) so as to extract a reference sample (E1) of a mineral of the at least one reference rock (R1 and free the argon-40 contained in the reference sample (E1), the treatment step consisting in firing (208) at the at least one reference rock (R1) with a laser (21) in order to ablate a reference sample (E1 of a mineral of the at least one reference rock (R1) and form a plasma from the sample (E1);

- determining (212) the number ($^{40}Ar_{measured}$*) of argon-40 atoms in the gases obtained from the plasma formed from the reference sample (E1) with the aid of analysis means (56);

- calculating (214) the mass (m*) of the reference sample (E1) from the age (t*) of the at least one reference rock (R1) from which it is obtained, from the nature (N*) of the mineral constituting the reference sample (E1) and from the number ($^{40}Ar_{measured}$*) of argon-40 atoms in the reference sample (E1);

- associating (216) the mass (m*) of the reference sample (E1) with the characteristics (D*, I*) of the fire effected by the laser (21) and with the nature (N*) of the mineral constituting the reference sample (E1).

FIG.1

# FIG.2

**FIG.3**

| 102 | Prélèvement de la roche R et introduction de la roche R prélevée dans le dispositif d'analyse |
|---|---|

| 104 | Mise sous vide de l'enceinte |
|---|---|

| 106 | Sélection d'un minéral à l'aide de la caméra et orientation du laser |
|---|---|

| 108 | Tir laser de D,I déterminées sur ce minéral |
|---|---|

| 110 | Détermination de la composition élémentaire de l'échantillon et de $k_{total}$ |
|---|---|

| 112 | Séparation des gaz et détermination de $^{40}Ar_{mesuré}$ |
|---|---|

| 114 | Transmission des résultats à la station de réception |
|---|---|

## FIG.4

202 — Prélèvement d'une roche d'âge connu

204 — Mise sous vide de l'enceinte

206 — Sélection d'un minéral et orientation du laser

208 — Tir laser de D*,I* déterminées sur ce minéral

212 — Séparation des gaz

Détermination de $^{40}Ar^*_{mesuré}$

214 — m* en fonction de t*, $^{40}K^*$, $^{40}Ar^*_{mesuré}$

216 — Stockage dans la base de données de m*, D*, I* et N*

## FIG.5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **SWINDLE.** AGE (Argon Geochronology expriment) : An Instrument for in situ Geochronology on the Surface of Mars. *Lunar and Planetery Science XXXIV,* 2003

- **FENNEMA et al.** A Method for Finding the Mass of a Milligram-Sized Rock Sample Without Using a Scale; with Possible Spacecraft applications. *Lunar and Planetary Science XXXVIII,* 2007